Europäisches Patentamt

European Patent Office

Office européen des brevets

(11) Publication number: 0 300 666
A1

## (12) EUROPEAN PATENT APPLICATION

(21) Application number: 88306367.9

(51) Int. Cl.⁴: C12M 3/04 , C12M 1/24

(22) Date of filing: 13.07.88

(30) Priority: 18.07.87 GB 8716997

(43) Date of publication of application:
25.01.89 Bulletin 89/04

(84) Designated Contracting States:
AT BE CH DE ES FR GR IT LI LU NL SE

(71) Applicant: Sterilin Limited
Lampton House Lampton Road
Hounslow Middlesex TW3 4EE(GB)

(72) Inventor: Kreuzer, Michael H.
63, Kingston Lane
Teddington, Middlesex(GB)
Inventor: Wickham, Jane M.
Combourne Farm
Goudhurst, Kent(GB)
Inventor: Walding, Dean
9 The Hollies Tyllwyd Parc
Quakers Yard Mid Glamorgan, Wales(GB)

(74) Representative: Dodd, David Michael et al
ROYSTONS 531 Tower Building Water Street
Liverpool L3 1BA(GB)

(54) Bulk cell culture vessel.

(57) A bulk cell culture vessel (10) comprising a generally cylindrical body part (12) and a separable end part (14) having, distinct from any closure cap provision for normal in-use access to the vessel, spaced peripheral formations (20, 22). One (20) enters the body part (12) and another (22) goes over the body part (12) with a seal (38) accommodation between those formations (20, 22). Said other peripheral formation (22) cooperates with exterior formation (32) of the body part (12) for the purposes of mutual securement and compression of a resilient seal (38) in said seal accommodation by an edge of the body part (12) going between the one (20) and the other (22) peripheral formations.

FIG.1

FIG.2

EP 0 300 666 A1

## Bulk Cell Culture Vessel

This invention relates to vessels having application for bulk culture of mammalian cells within them, particularly generally cylindrical vessels.

It is intended that vessels hereof should be capable of use in the manner of roller bottles, but not necessarily limited to such use; also that they can accommodate means presenting high surface area to facilitate culturing of cells that require to adhere to a surface, such as spiral cylindrical units formed of sheet material with a corrugated spacer, see British patent specification No. 1376131; but also not necessarily be so limited.

Our prior implementation of a cylindrical vessel generally as in the above mentioned British patent specification had disadvantages compared with proposals hereof. A particular disadvantage of the prior vessel arose from it being made as two halves affording and extending from ends of the vessel and welded together at a transverse seam substantially centrally of the length of the cylindrical vessel. Those halves were readily made with integral internal spacers/locaters contacting and entering the spiral unit, and the resulting vessel is satisfactory in terms of ease of assembly with a spiral cylindrical unit located therein, and supply in a sterile state. However, the vessel can not readily be opened up for detailed inspection of the spiral unit after use in culturing cells introduced along with suitable nutrient medium. Obvious alternatives affording releasable connection of the vessel halves, including taping over and/or interfitting edge formations have been considered but not found to be satisfactory at least in terms of physical integrity and strength, if not further as to contamination risks.

Also, difficulties were encountered when considering alternative constructions having at least one if not both ends as removable caps, both in terms of unsuitability for cell culturing requirements of conventional cap connection and sealing arrangements and in terms of making satisfactory integral location provisions for a spiral cylindrical unit. The present invention solves such difficulties and results in a vessel that is simple to use and of inherently great flexibility of application.

According to one aspect of this invention, there is provided a vessel suitable for cell culture therein, the vessel comprising a generally cylindrical body part and an end part having, distinct from any closure cap provision, one formation that enters the body part and another formation that goes over the body part and a seal accomodated between those formations, said other formation cooperating with exterior formations of the body part for the purposes of mutual securement and compression of the seal by an edge of the body part going between the one and the other formations. Cooperating formations of the body part, say as an abutment, and of the end part, say of its said other formation, may usefully cooperate to limit relative movement of the parts during securement and to assure optimising of seal action.

It is preferred that said one formation of the end part and the end of the body part into which it is entrant conform closely to each other in a range from actual interference contact to a small spacing (say up to 1 millimetre) having capillary characteristics relative to nutrient medium so as to render static any such medium entering that spacing. Then, the seal is effectively isolated from cell culturing activity within the vessel.

At least then, mutual screw-threading can serve for securement purposes, as could bayonet-type or cam-type cooperating formations, and there is no need of close internal and external diameter requirements of the interior of said other formation of the end part and of the exterior of the body part, respectively, where they overlap and afford securement formations.

It is, of course, feasible for such provisions for releasably engaging parts of cell culture vessels to be made at both ends, i.e. for two end parts, or even applied at the juncture of two body halves previously mentioned for our prior cell culture vessel at least as further modified to have the preferred location provisions hereof for an inner unit.

Suitable location provisions for an inner unit also of cylindrical shape, at least at its ends, can comprise locaters, or locater formations if integral, associated both with the end part and with the body at a closed end thereof (or another end part), which locater formations engage only ends and exterior end-adjacent sides of an inner unit, thereby accurately to space that unit from vessel ends and sides as desired or required. Then, at least relative to a spiral cylindrical inner unit, any requirements for relative turning of the cell culture vessel parts in their mutual securement will not damage the inner unit.

An end part with integral locater formations can have the latter protrude from the end part proper and taper or curve down to their free ends or edges so as to aid insertion between interior and exterior surfaces of the body part and the inner unit, respectively. Both of the latter can conveniently terminate substantially in the same plane transversely of the cylindrical vessel, at least taking account of an end baffle for the inner unit, which baffle may further have formations to engage between turns of a spiral said inner unit.

An alternative is for locater formations engaging sides of the end part adjacent end of the inner body to be on a baffle member disposed between the inner body and end-facing (relative to the inner body) locater formations of the end part.

Outward doming of the blind end of the cell culture vessel allows within a longer-extending skirt the vessel to be stood on that end in a stable manner, in which attitude it can, of course, readily be drained by a suction tube or gassed from below the inner unit.

At least then, it is preferred that said end-part of the cell culture vessel be of a nature permitting entry of nutrient medium and cells to be cultured, also any associated draining/gassing tubes, etc. Accordingly, preferred said end-parts shoulder outwardly to a neck that may also be suitably threaded for a closure cap in the as-supplied sterile condition. In or for use, such closure cap will be removed at least for entry of nutrient medium and cells to be cultured, and can be replaced by an entry system fitting to the neck and further serving for gassing, draining, etc tubes and connections.

Specific implementation of one embodiment of this invention will now be described, by way of example, with reference to the accompanying drawing, in which:-

Figure 1 is a side view of a cell-culture vessel 10, partly broken away to sections;

Figure 2 is an enlarged detail sectional view from A of Figure 1, and showing mutual securement provisions for body and end parts 12 and 14, respectively, by threading;

Figure 3 is an enlarged detail sectional view from B of Figure 1, specifically of the end part 14;

Figure 4 is an enlarged detail sectional view from C of Figure 1, specifically of the closed or blind end of the body part;

Figure 5 is an end view of the closed or blind end of the body part on the arrow C of Figure 1;

Figure 6 is a side view of another cell-culture vessel 110, partly broken away;

Figures 7A and 7B are top and bottom plan views of its end part;

Figures 8A, 8B and 8C are enlarged section, and side and detail views for one of cooperating bayonet-type formation;

Figure 9 is a top plan view of the vessel 110;

Figures 10 and 10B, 10C and 10D are side and enlarged section, side and detail views of the other of cooperating bayonet-type formations; and

Figures 11A and 11B are plan and section views of a preferred baffle member.

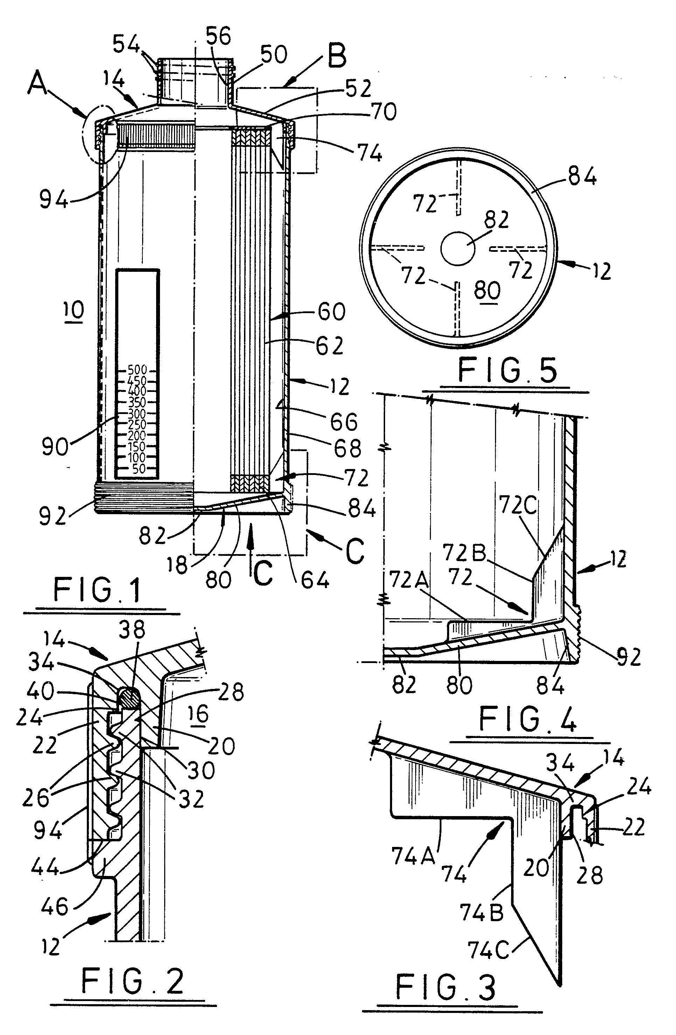

In Figures 1 to 5, the cell culture vessel 10 is of generally cylindrical shape with the end part 14 closing off the body part 12 at its open end or mouth 16. The other end 18 of the body part 12 is shown blind or closed off.

The end part 14 has a depending rim comprised of two spaced wall formations 20 and 22, the inner one of which (20) enters the mouth 16 of the body part 12 and the other or outer one of which (22) goes about the body part adjacent its open end or mouth 16.

The outer wall 22 is shown longer than the inner wall 20 and has its interior surface 24 effectively relieved to leave a screw thread formation 26. The inner wall 20 enters the body part 12 through its mouth to and is a close fit at its exterior surface 28 to the interior surface 30 past the mouth 16 of the body part 12, whether touching (shown) or at close spacing (not shown) then with capillary characteristics for nutrient medium used in cell culturing. The body part 12 has an exterior screw-thread formation 32 adjacent to its open end or mouth 16, and the thread formations 26 and 32 match each other for mutual securement of the parts 12 and 14.

At its blind innermost part, beyond the thread formation 26, the circular space between the walls 20 and 22 affords accommodation 34 for a resilient seal 38 shown, as being of simple O-ring type. The seal 38 is sealingly engaged, in fact will be compressively distorted (not shown) by the edge 40 of cylindrical side walling 42 of the body part 12, i.e. at the lip of the mouth 16. Such compressive distortion is limited for maximum effectiveness in terms of sealing by the fact that free end 44 of the outer wall 22 meets abutment 46 shown as an exterior rib on the body part 12.

The end part 14 is shown with a neck portion 50 disposed centrally on an outwardly sloping shoulder formation 52 from its rim that affords the depending walls 20 and 22 . Outer threading 54 of the neck portion indicates connection for a closure cap (not shown) at first supply of the cell culture vessel 10, usually in a sterile condition. In use, of course, such closure cap can be removed to load the vessel 10 with the nutrient medium and cells to be cultured. Some suitable fitting to the neck portion 50 may be used for that purpose and/or for gassing and/or draining tubes etc. Alternatively, a special closure cap could be used.

The cell culture vessel 10 is shown accommodating an inner unit 60 which is shown for convenience as being of the above-mentioned spiral cylindrical form. Specifically, a sheet 62 of cell-adherent usually plastics material is rolled up with successive turns spaced, see spacers 64 which may conveniently be of a corrugated strip material. Rolling up is from an inner diameter shown generally corresponding to the interior diameter (56) of the neck portion 50 of the end part 14 to an outer diameter spaced from inner surface 66 of cylindrical walling 68 of the body part 12. A flat ring-like baffle 70 is shown against that end of the unit 60 adjacent to the neck portion 50 of the end part 14.

Locaters for the unit 60 are shown as interior fins 72 at the blind end of the body part 12, and as fins 74 at the end part 14 that are both interior and protrude from that end part 14. Both of fins 72 and pins 74 have relatively perpendicular surfaces 72A, 74A and 72B, 74B to engage ends and end-adjacent sides, respectively of the unit 60. Further surfaces 72C, 74C taper away from the side engaging surfaces 72B, 74B to assist assembly. Specifically, tapering surface 72C assists centering the unit 60 in the body part 12, and tapering surfaces 74C assist both confirming such centralising of the unit 60 and training the end part 14 onto the body part 12.

The blind end 18 of the body part 12 is outwardly domed at 80, actually to a central flat 82, surrounded by a skirt 84 of slightly greater extent. The skirt 84 enables the cell culture vessel to be stood stably on its blind end 18, and the doming 80 assures that the cell-culture vessel 10 can readily be drained by tube. The skirt 84 is shown as extension of side-walling 68 of the body part 12, but could obviously be otherwise located.

Graduations are indicated at 90 for assisting loading of the cell-culture vessel 10 with nutrient medium whilst in an upright position. Normally, the nutrient medium will be in an amount insufficient to re-enter the neck portion 50 when the cell culture vessel is laid on its side, say for rolling, so the closure cap (not shown) and neck portion 50 need not have similar engagement provisions as for the body and end parts 12 and 14, but it could have if desired or required.

In connection with assisting rolling (when used as a roller bottle), grip-assist formations can be made on the cell-culture vessel 10, see at 92 on the body part 12 and at 94 on the outer surface of the outer wall 22 of the end part 14. Alternatively, such formations may be later applied, say as textured tape or elastic bands. Oppositely directed parallel formations may assist holding the cell culture vessel 10 steady on a rolling mechanism, i.e. militate against axial translation.

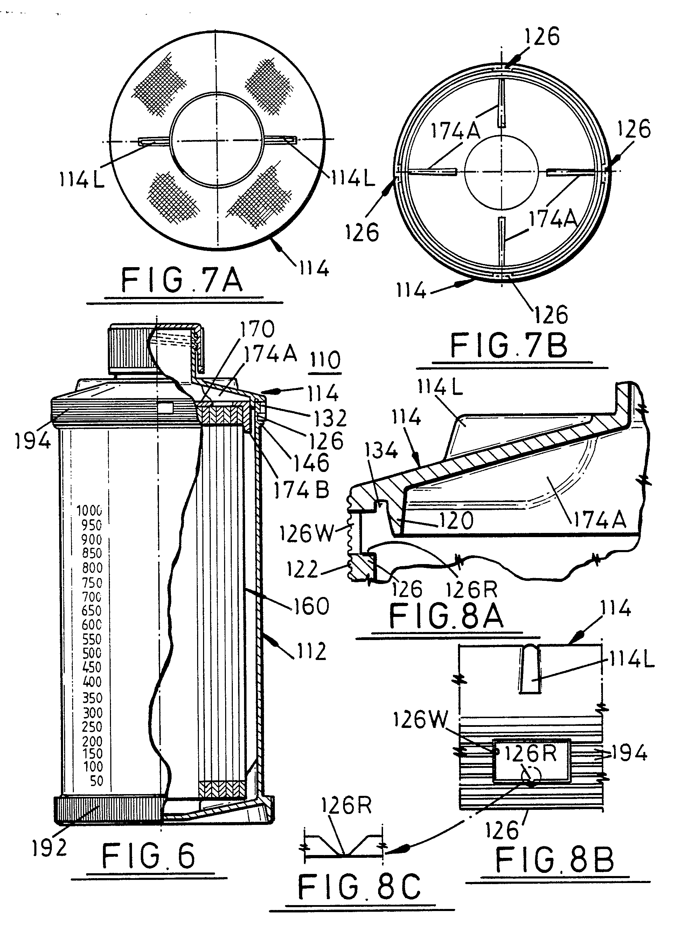

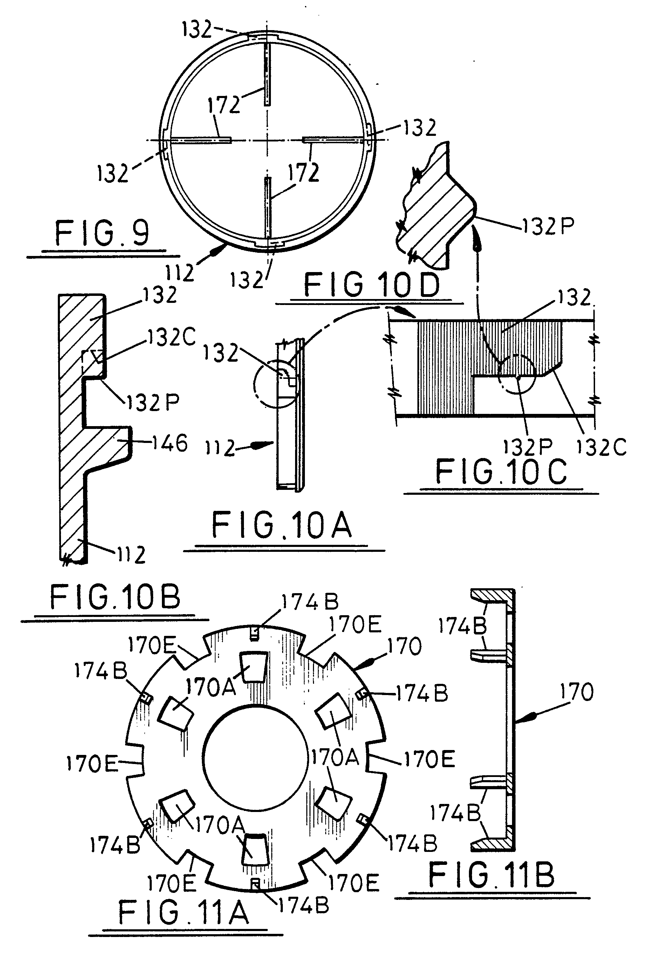

Turning to Figures 6 to 11, the cell culture vessel 110 differs from that of Figures 1 to 5 in its use of bayonet-type securements for its end part 114 and its body part 112. Each such bayonet securement comprises cooperating lugs, one (126) as an interior projection on outer wall 122 of the end part 114, and the other (132) as an exterior formation on the mouth of the body part 112. Four such securement formations are shown symmetrically disposed, such number and locations being preferred but not essential.

Each of the end-part lugs 126 is below a window 126W through the outer wall 122 and has a small recessing 126R as a slot shown central thereof across its thickness, conveniently formed by mould tool parts entrant the window 126W. Each of the body part lugs 132 will engage over a corresponding one of the end part lugs 126 passing its end chamfer 132C, and has a small pip 132P shown substantially central thereof, preferably extending as a ridge across its thickness. The pip 132P will engage in the recess 126R with a snap action at securement of the end part 114 to the body part 112, and can be checked through the windows 126W.

Another difference compared with Figures 1 to 5 concerns the baffle 170, here shown with depending fins or teeth 174B serving to engage end-adjacent sides of the inner unit 160, i.e. the same purpose as parts 74B of locater fins 74 in Figures 1 to 5. Such fins of the embodiment of Figures 1 to 7 are then restricted effectively only to fins 174A equivalent to fin parts 74A.

The baffle 170 is further shown with edge slots 170E and apertures 170A medial of its ring width serving to aid controlled loading unloading, also circulation, of nutrient medium in the vessel 110 and its inner unit 160.

Also, the end part 114 is shown with exterior lugs or fins 114L as finger grips to aid its securement to the body part 112; and the directions of the grip assist formations 192 and 194 are reversed compared with Figure 1.

In general, the accompanying Figures show a vessel particularly suited to use as a roller bottle. In doing so, and in showing a single central entry (capped in use) to the end part 14 or 114, it is not intended that other use of the vessel be excluded, nor that entry provisions via the end part 14 or 114 be limited to a single central part. Thus, a two or three part entry could be provided, say via a variant end part, though similarly to the body part 12 or 112. Such multiple port vessels serve particularly for separate input of nutrient medium and a suitable microbiological activity enhancing gas, also for exit of waste gas.

## Claims

1. A bulk cell culture vessel comprising a generally cylindrical body part and a separable end part having, distinct from any closure cap provision for normal in-use access to the vessel, spaced peripheral formations one that enters the body part and another that goes over the body part and a seal accommodation between those formations, said other peripheral formation cooperating with exterior formation of the body part for the purposes of mutual securement and compression of a resilient seal in said seal accommodation by an edge of the body part going between the one and the other peripheral formations.

2. A bulk cell culture vessel according to claim 1, wherein cooperating formations of the body part and of the end part serve to limit relative movement of those parts during mutual securement.

3. A bulk cell culture vessel according to claim 2, wherein the cooperating formations comprise an abutment formation of the body part and said other peripheral formation of the end part.

4. A bulk cell culture vessel according to any preceding claim, wherein said one formation conforms closely to its entry into an end of the body part in a range from contact to a spacing having capillary characteristics in relation to nutrient medium for the vessel.

5. A bulk cell culture vessel according to any preceding claim, wherein said other peripheral formation of the end part and said exterior formation of the body part cooperate for securement purposes as screw threadings.

6. A bulk cell culture vessel according to any preceding claim, comprising locaters for an inner unit, the locaters being associated with at least one end of the vessel and engaging a said inner unit only at an end and an exterior end-adjacent part of that inner unit.

7. A bulk cell culture vessel according to claim 6, wherein said locaters are on the end part and have protrusions beyond the peripheral formations of the end part to enter the body part between its interior surface and a said inner unit.

8. A bulk cell culture vessel according to claim 6, wherein card locaters are partly on an apertured baffle member fitting over a said inner unit adjacent the end part and partly on the end part to engage said baffle member.

9. A bulk cell culture vessel according to any preceding claim, wherein a blind end of the body part opposite the end part is outwardly domed within a longer extending skirt on which the vessel can be stood upright.

10. A bulk cell culture vessel according to any preceding claim, wherein the end part has an access entry interfitting at least with a closure cap.

EP 0 300 666 A1

FIG.1

FIG.2

FIG.3

FIG.4

FIG.5

FIG.7A

FIG.7B

FIG.6

FIG.8A

FIG.8B

FIG.8C

FIG.9

FIG 10 D

FIG.10 C

FIG.10 A

FIG.10 B

FIG.11A

FIG.11B

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 381 103 (INSTITUT PASTEUR DE LILLE)<br>* Figure; claims * | 1-5,10 | C 12 M   3/04<br>C 12 M   1/24 |
| Y | | 6-8 | |
| A | US-A-4 665 035 (J. TUNAC)<br>* Figures * | 6-8 | |
| Y,D | GB-A-1 376 131 (NATIONAL RESEARCH DEVELOPMENT CORP.)<br>* Figures 8,9; page 4, lines 66-97 * | 6 | |
| Y | US-A-4 317 886 (L.R. JOHNSON et al.)<br>* Figures 1,2 * | 6-8 | |
| A | US-A-4 337 104 (R.W. LYNN)<br>* Figures; claims * | 1 | |
| A | US-A-3 708 400 (A. HIRSCH) | | |
| X | DE-A-3 218 671 (R. MÜLLER)<br>* Figures * | 1,4,5 | TECHNICAL FIELDS SEARCHED (Int. Cl.4) |
| | | | C 12 M |

The present search report has been drawn up for all claims

| Place of search | Date of completion of the search | Examiner |
|---|---|---|
| THE HAGUE | 24-10-1988 | COUCKE A.O.M. |

EPO FORM 1503 03.82 (P0401)